# EUROPEAN PATENT APPLICATION

(11) **EP 2 181 694 A1**
(43) Date of publication of application: **05.05.2010**
(21) Application number: 08305747.1
(22) Date of filing: 29.10.2008
(51) Int. Cl.: A61K 8/31, A61K 8/39, A61K 8/90, A61Q 19/00

(54) **Water free rinse-off skin-conditioning composition**

(71) Applicant: Johnson & Johnson Consumer France SAS, 92130 Issy-les-Moulineaux (FR)
(72) Inventor: Sans, Anne, 27400, Amfreville Sur Iton (FR); Candolives-Marchais, Marjorie, NJ, 08536 (US); Cros-Lefebvre, Marjorie, 27400, Houetteville (FR)
(74) Representative: Warcoin, Jacques

(57) **Abstract**

The present invention relates to a water free rinse-off skin-conditioning composition comprising:
(a) 50-95% by weight of two or more oil(s), one of the oils being a straight or branched hexadecane,
(b) 3-10% by weight of one or more oil thickener(s), and
(c) 4-20% by weight of one or more nonionic surfactant(s),
as well as to a method of moisturizing skin comprising applying such a composition.

## Description

The invention relates to a moisturizing rinse-off skin-conditioning composition, which has to be used on wet skin while showering, then rinsed off and finally toweled dry, and to a method of moisturizing skin.

Showering can dry the skin and decrease the lipid barrier. Usually, the application on the skin of a moisturizing lotion after the shower allows to compensate it. However, in order to avoid the drying effect after the shower, it will be preferable to moisturize the skin while still being under the shower. For this, the moisturizing product has to be able to mix easily with water in order to be rinsed off without problems after application under the shower, while having a good moisturizing capacity and a pleasant feel after application to the consumer and not a greasy after feel.

Such moisturizing rinsed-off compositions have already been described in the prior art. Most of them are in the form of an emulsion or of liquid oils which are runny into the hands while showering and thus not at all practical. But the sensorial experience while applying the product is not satisfying. It is not visually clear where the product has been applied and if enough has been used.

US 5,888,492 described a rinse-off skin-conditioning composition which is neither an emulsion, nor strictly oily and which comprises a mineral oil as softening agent, a mixture of copolymers di-block and tri-block as thickener, a fatty acid ester as emollient and a surfactant. However, an unpleasant sticky after feel is observed after its application.

The inventors have thus surprisingly found that the use of an hexadecane, such as isohexadecane, in combination with other mineral oil(s), oil thickener(s) and surfactant(s) allows to obtain a composition with high performances of moisturizing, optimized oils rinsability and good skin feel with no greasy or sticky after feel.

Therefore, the invention pertains to a skin-conditioning composition which is designed to be applied on wet skin (but not immersed in water), notably in the shower, and rinsed off by showering, leaving a skin-conditioning deposit on the skin's surface which is aesthetically acceptable, not greasy or sticky.

The present invention concerns in particular a water free rinse-off skin-conditioning composition comprising:
(a) 50-95%, preferably 60-85%, more preferably 80-85%, by weight of two or more oil(s) relative to the total weight of the composition, one of the oils being a straight or branched hexadecane, ,
(b) 3-10%, preferably 5-7%, more preferably 5-6%, by weight of one or more oil thickener(s) relative to the total weight of the composition, and
(c) 4-20%, preferably 4-10%, more preferably 5-7%, by weight of one or more nonionic surfactant(s) relative to the total weight of the composition.

Advantageously, the composition contains from 3 to 50%, preferably from 3 to 10%, by weight of the total composition of a straight or branched hexadecane as one of the oils.

The "straight or branched hexadecane" is preferably isohexadecane.

When applied to the skin while showering, the composition of the invention is easily rinsed off with water due to its miscibility with water and forms a nice white creamy film on the skin while mixed with water. It delivers good moisturizing properties despite the fact that it is rinsed off, without leaving an unpleasant greasy or sticky skin feel, due to excessive deposit of the product remaining on the skin after showering.

The "two or more oils" are used as a skin-conditioning agent in order to moisturize and soften the skin and thus must be contained in sufficient quantities such that a residue of the oil is deposited after rinsing, to effect skin conditioning.

Within the framework of the present invention, an oil means preferably an apolar oil such as an hydrocarbon oil, optionally in association with a vegetable oil.

A hydrocarbon oil such as mineral oil can be in particular a straight or branched, preferably straight, chain hydrocarbon, of 10 to 200, preferably 10 to100, more preferably 10 to 40, carbon atoms. Such a hydrocarbon oil can be natural or synthetic but natural oils are generally preferred. Examples of mineral oils are liquid paraffin, polydecenes, polyisobutenes, hydrogenated polyisobutenes, squalane and squalene.

Preferably, the two or more oils are a mixture of liquid paraffin and a straight or branched hexadecane, and more preferably a mixture of liquid paraffin and isohexadecane.

The "one or more oil thickener(s)" allows to increase the oil viscosity, as mineral oils have generally a low viscosity, and to avoid the product to run off the hand through fingers and also to run off the body when applied.

Increasing the viscosity of hydrocarbon oils, as used advantageously in the present invention, in a composition suitable for topical application is however quite difficult. The thickener has to increase sufficiently the viscosity to avoid to obtain a liquid product but, in the same time, the product has to be still pourable and not transform in a paste or a gel. There are relatively few inexpensive technologies available to thicken in such a manner a non-polar oil system such as mineral oil.

The patent US 5,221,534 is directed to mineral oil gels wherein the mineral oil, including mineral oil suitable for use in the present invention, are "gelled" by the addition of di-block and/or tri-block copolymers comprised of styrenic monomers and low glass transition temperature (Tg) monomers, such as butadiene and isoprene. Even if the aim of US 5,221,534 is to provide mineral oil gels and not pourable composition, the same copolymers can be employed in the present invention, but with lower quantities of polymer relative to the quantities used in US 5,221,534 as the viscosity requirements are different in the present invention and in US 5,221,534.

In particular, suitable di-block and tri-block copolymers are di-block and tri-block copolymers based on styrene and isoprene or styrene and butadiene, or hydrogenated di-block or tri-block copolymers based on styrene and ethylene/butylene or styrene and ethylene/propylene. Particularly preferred are the hydrogenated di-block and tri-block copolymers based on styrene and ethylene/propylene and preferably a hydrogenated styrene-ethylene/propylene-styrene diblock copolymer.

The term "styrenic" or "styrene" refers, in the context of the present invention, to styrene as well as to substituted styrene such as ortho-, meta- and para-alkyl styrene, where "alkyl" means a straight or branched hydrocarbon chain comprising 1 to 3 carbon atoms. Preferably, it refers to a non-substituted styrene.

The use of such thickener presents the further advantage to improve the skin-conditioning effect of the mineral oil(s).

The addition of a non-ionic surfactant is a required element for the rinse-off skin composition of the invention to ensure that the product is rinsable. The surfactant must be both soluble in the oil(s) and effective in dispersing the formula into water, in order to achieve effective rinsing off. At the same time, if effectively 100 percent of the product is rinsed away, little or no skin-conditioning effect will be attained. Thus, amounts substantially in excess of 20 percent by weight of the total composition, as applied, are aesthetically-undesirable to users, while amounts substantially below about 4 percent provide no perceptible effect.

Suitable nonionic surfactants can be selected in particular in the group consisting of Laureth-2, Laureth-3, Laureth-4, Oleth-3, Isosteareth-2, Trideceth-3, C9-11 Pareth-3, C11-15 Pareth-3, C12-13 Pareth-3, C11-15 Pareth-5, PEG-40 Sorbitan Peroleate, PEG-40 Sorbitan Hexaoleate, PEG-50 Sorbitan Hexaoleate, Steareth-2, cocamide MEA, lauramide MEA, mystiramide MEA, Polysorbate-21 and mixtures thereof (PEG = poly(ethylene glycol), and MEA = monoethanolamine). Preferably, the non ionic surfactant(s) is laureth-4.

Advantageously, the composition comprises a mixture of liquid paraffin and isohexadecane as oil(s), a hydrogenated styrene isoprene copolymer as oil thickener(s) and laureth-4 as nonionic surfactant(s).

In order to achieve the desired properties of moisturizing, rinsability and the like of the composition, the physico-chemical characteristics of the composition including viscosity, water dispersibility, hydrating potential and the like must be balanced in the composition.

The best combination of the essential components of the composition has been achieved with a ratio oil(s)/oil thickener(s)/nonionic surfactant(s) of 15.25/1/1.18.

The composition of the invention can also comprise one or more additive(s), such as antimicrobial agents, antioxidants, dermatologically active agents, emollients, other humectants, other thickening agents, fragrances, preservatives, pigments or colorants or opacifiers.

These additional additives will be incorporated in limited weight amounts so as not to interfere with the viscosity, moisturizing and rinse-off characteristics of the base composition. Such additives are conventional to those of skill in the art.

Examples of these additives are listed below as well as in the International Cosmetic Ingredient Dictionary and Handbook, eds. Wenninger and McEwen (The Cosmetic, Toiletry, and Fragrance Assoc., Washington, D.C., 7@th Edition, 1997) (hereinafter "ICT Handbook").

Antimicrobial agents can be used when the composition is to be applied to skin prone to microbial infection, e.g., by bacteria, fungal, or protozoa. Examples of such agents include benzyl alcohol, chlorobutanol, phenylethyl alcohol, phenylmercuric acetate, potassium sorbate, sorbic acid, benzoic acid, phenoxyethanol, butylparaben, ethylparaben, methylparaben, propylparaben, and sodium benzoate, and in particular methylparaben. Other antimicrobial agents are listed on page 1612 of the ICT handbook.

Antioxidants can be used to protect ingredients of the composition from oxidizing agents that are included within or come in contact with the composition. Examples of antioxidants include ascorbyl palmitate, butytlated hydroxyanisole, butylated hydroxytoluene, potassium propyl gallate, octyl gallate, dodecyl gallate, phenyl-α-napthyl-amine, and tocopherols such as α-tocopherol. Other antioxidants are listed on pages 1612-13 of the ICT Handbook.

Dermatologically active agents include agents for treating wound healing, inflammation, acne, psoriasis, cutaneous aging, skin cancer, impetigo, herpes, chickenpox, dermatitis, pain, itching or skin irritation. Examples of such dermatologically active agents include hydrocortisone, dexamethesone, panthenol, phenol, tetracycline hydrochloride, yeast, hexylresorcinol, lamin, kinetin, betamethasone, triamcinolone, fluocinolone, methylprednisolone, retinoids such as retinol and retinoic acid, dapsone, sulfasalazine, resorcinol, salicylic acid, benzoyl peroxide, erythromycin-benzoyl peroxide, erythromycin, clindamycin, mupirocin, griseofulvin, azoles such as miconazole, econozole, itraconazole, fluconazole, and ketoconazole, ciclopirox, allylamines such as naftifine and terfinafine, acyclovir, famciclovir, valacyclovir, benzocaine, lidocaine, dibucaine, pramoxine hydrochloride, methyl salicylate, camphor, menthol, resocinol, and vitamins such as tocopherol, and tocopherol acetate.

Emollients are agents that soften and smooth the skin. Examples of emollients include oils and waxes such as microcrystaline wax, polyethylene, triglyceride esters such as those of castor oil, cocoa butter, safflower oil, corn oil, olive oil, cod liver oil, almond oil, palm oil, squalene, and soybean oil, acetylated monoglycerides, ethoxylated glycerides, fatty acids, alkyl esters of fatty acids, alkenyl esters of fatty acids, fatty alcohols, fatty alcohol ethers, ether-esters, lanolin and derivatives of lanolin, polyhydric alcohol esters, wax esters such as beeswax, vegetable waxes, phospholids, and sterols, isopropyl palmitate or glyceryl stearate, and in particular almond oil or fatty alcohols such as cetyl, stearyl and/or myristyl alcohols. Other emollients are listed on pages 1656-61 of the ICT handbook.

Siloxane are particularly preferred emollient. Siloxanes that may be used in the present invention include, but are not limited to, dimethicone, cyclomethicone, phenyl trimethicone, phenyl dimethicone, cetyl dimethicone, stearyl dimethicone, amodimethicone, C₃₀₋₄₅ alkyl dimethicone, C₃₀₋₄₅ Alkyl Methicone, Cetearyl methicone, dimethicone copolyol, cyclopentasiloxane, cyclohexasiloxane or any combinations thereof. In particular, amodimethicone could be used as emollient in the present invention.

Additionnal thickening agents could be in particular fatty alcohols such as cetyl, stearyl and/or myristyl alcohols.

Examples of fragrances or perfume include peppermint, rose oil, rose water, aloe vera, clove oil, menthol, camphor, eucalyptus oil, and other plant extracts. To eliminate certain odors from compositions, masking agents may be used. Other fragrances and masking agents are listed on pages 1639-40 of the ICT Handbook.

Preservatives can be used to protect the composition from degradation. Examples of preservatives include phenoxyethanol, methylparaben, benzalkonium chloride, benzethonium chloride, propyl paraben, benzoic acid, benzyl alcohol, and mixtures thereof such as liquipar oil. In particular, it can be phenoxyethanol, methylparaben or a mixture thereof. Other preservatives are listed on pages 1654-55 of the ICT Handbook. However, the composition of the present invention can be preservative free.

Pigments or colorants are used to modify the color of the composition, such as to obtain a white composition. It can be in particular titanium dioxide.

Opacifiers, such as titanium oxide, are used in clear or transparent composition in order to render it opaque. The present invention can thus be clear or opaque according to the use or not of an opacifier.

According to a particular embodiment, the composition consists of:
1) 60-90% of Liquid paraffin
2) 3-10% of Isohexadecane
3) 5-7% of a Hydrogenated Styrene-ethylene/propylene-styrene diblock Copolymer
4) 0-4% of Cetyl Alcohol, Stearyl Alcohol, Myristyl Alcohol
5) 0-1% of Titanium Dioxide
6) 0-2% of Amodimethicone
7) 4-10% of Laureth-4
8) 0-1% of Fragrances
9) 0-1% of Phenoxyethanol
10) 0-1% of Methylparaben
11) 0-1% of Prunus Dulcis

The presence of Prunus Dulcis in the composition is particularly suitable for dry to very dry skins.

The present invention concerns also a method of moisturizing skin comprising applying a composition of the invention as described above on a wet skin while showering and rinsing off.

The compositions of the invention are prepared by simple blending of the various formulation components, pursuant to art-recognized technology. However, certain ingredients may require limited heating. Under the current state of the law, the method of preparation, per se, of the inventive composition does not constitute an aspect of the invention.

The following non-limiting examples further illustrate the invention.

### EXAMPLE 1

The compositions of the inventions have in particular the following composition (in weight %):
1) 80-85% of Liquid paraffin
2) 3-10% of Isohexadecane
3) 5-7% of Hydrogenated Styrene-ethylene/propylene-styrene diblock Copolymer
4) 0-4% of Cetyl Alcohol, Stearyl Alcohol, Myristyl Alcohol
5) 0-1% of Titanium Dioxide
6) 0-2% of Amodimethicone
7) 4-10% of Laureth-4
8) 0-1% of Fragrances
9) 0-1% of Phenoxyethanol
10) 0-1% of Methylparaben
11) 0-1% of Prunus Dulcis

The compositions of the invention have been prepared according to the following steps:
1- Heating at 75°C of liquid paraffin,
2- Addition of isohexadecane under stirring and still at 75°C,
3- Addition of the hydrogenated styrene-ethylene/propylene-styrene diblock copolymer at 60-70°C,
4- Addition of titanium dioxide and of the fatty alcohols (cetyl, stearyl and myristyl alcohols) if any after complete dissolution of the hydrogenated styrene-ethylene/propylene-styrene copolymer,
5- Cooling at 35°C,
6- Addition of amodimethicone if any bellow 60°C,
7- Preparation of a premix of laureth-4, parabens and phenoxyethanol, if any, at 60°C,
8- Addition of the premix in the composition obtained at step 6, and
9- Addition of the perfume below 40°C.

### EXAMPLE 2

The following table gives the composition of four skin-conditioning compositions (A, B, C and D) with different quantities of isohexadecane.

| | INCI Name | A (%) | B (%) | C (%) | D (%) |
|---|---|---|---|---|---|
| 1 | Liquid paraffin | 83.00 | 88.90 | 49.00 | 80.00 |
| 2 | Isohexadecane | 3.90 | - | 39.90 | 3.90 |
| | Hydrogenated Styrene- | 5.00 | 5.00 | 5.00 | 5.50 |
| 3 | ethylene/propylene-styrene diblock Copolymer | | | | |
| 4 | Cetyl Alcohol, Stearyl Alcohol, Myristyl Alcohol | 2.00 | - | - | 2.00 |
| 5 | Titanium Dioxide | 0.30 | 0.30 | 0.30 | 0.30 |
| 6 | Amodimethicone | 1.00 | 1.00 | 1.00 | 1.00 |
| 7 | Laureth-4 | 4.00 | 4.00 | 4.00 | 6.50 |
| 8 | Fragrances | 0.80 | 0.80 | 0.80 | 0.80 |

### Sensorial test:

These four compositions have been tested on 6 panellists according to the following protocol:
1. Wash your body with your usual product and rinse it off,
2. Still under the shower, pour the composition A, B, C and D in your hand and spread it on your wet skin,
3. Rinse it off, and
4. Dry your body

After the use of the 4 compositions, each panellist has thus to determine which composition, among compositions A, B, C and D, has the easiest application and the easiest rinsing and which composition is the stickiest.

The results are presented on the table below:

| | Composition with the easiest application | Composition with the easiest rinsing | The stickiest composition |
|---|---|---|---|
| Panellist 1 | equal | equal | B |
| Panellist 2 | A, B and C | equal | B |
| Panellist 3 | equal | equal | B |
| Panellist 4 | B and C | equal | B |
| Panellist 5 | equal | equal | B and D |
| Panellist 6 | A | equal | B |

It appears thus that the composition B, which does not contain isohexadecane, is always felt as the stickiest composition.

## Claims

1. A water free rinse-off skin-conditioning composition comprising:
(a) 50-95%, preferably 60-85%, more preferably 80-85%, by weight of two or more oil(s), one of the oils being a straight or branched hexadecane, relative to the total weight of the composition,
(b) 3-10%, preferably 5-7%, more preferably 5-6%, by weight of one or more oil thickener(s) relative to the total weight of the composition, and
(c) 4-20%, preferably 4-10%, more preferably 5-7%, by weight of one or more nonionic surfactant(s) relative to the total weight of the composition.

2. A composition according to claim 1, **characterized in that** it contains from 3 to 50%, preferably from 3 to 10%, by weight of the straight or branched hexadecane relative to the total composition.

3. A composition according to claim 1 or 2, **characterized in that** the one or more oil(s) is a mixture of liquid paraffin and a straight or branched hexadecane.

4. A composition according to claims 1 to 3, **characterized in that** the straight or branched hexadecane is isohexadecane.

5. A composition according to any one of claims 1 to 4, **characterized in that** the one or more oil thickener(s) is a hydrogenated styrene-ethylene/propylene-styrene diblock copolymer.

6. A composition according to any one of claims 1 to 5, **characterized in that** the one or more nonionic surfactant(s) is laureth-4.

7. A composition according to any one of claims 1 to 6, **characterized in that** the composition comprises a mixture of liquid paraffin and isohexadecane as oil(s), a hydrogenated styrene-ethylene/propylene-styrene diblock copolymer as oil thickener(s) and laureth-4 as nonionic surfactant(s).

8. A composition according to any one of claims 1 to 7, **characterized in that** the ratio oil(s)/oil thickener(s)/nonionic surfactant(s) is 15.25/1/1.18.

9. A composition according to any one of claims 1 to 8, **characterized in that** the composition comprises further one or more additive(s), such as antimicrobial agents, antioxidants, dermatologically active agents, emollients, other humectants, other thickening agents, fragrances, preservatives, pigments or colorants or opacifiers.

10. A composition according to any one of claims 1 to 9, **characterized in that** the composition consists of:
1) 60-90% of Liquid paraffin
2) 3-10% of Isohexadecane
3) 5-7% of Hydrogenated Styrene-ethylene/propylene-styrene diblock Copolymer
4) 0-4% of Cetyl Alcohol, Stearyl Alcohol, Myristyl Alcohol
5) 0-1% of Titanium Dioxide
6) 0-2% of Amodimethicone
7) 4-10% of Laureth-4
8) 0-1% of Fragrances
9) 0-1% of Phenoxyethanol
10) 0-1% of Methylparaben
11) 0-1% of Prunus Dulcis

11. A method of moisturizing skin comprising applying a composition according to any one of claims 1 to 10 on a wet skin while showering and rinsing off.
